(19) Europäisches Patentamt
European Patent Office
Office européen des brevets

(11) Veröffentlichungsnummer: **0 102 327**
**A2**

(12) # EUROPÄISCHE PATENTANMELDUNG

(21) Anmeldenummer: **83810374.5**

(22) Anmeldetag: **22.08.83**

(51) Int. Cl.³: **C 07 D 239/62**
A 61 K 31/515

(30) Priorität: **26.08.82 CH 5080/82**

(43) Veröffentlichungstag der Anmeldung:
**07.03.84 Patentblatt 84/10**

(84) Benannte Vertragsstaaten:
**BE CH DE FR IT LI NL SE**

(71) Anmelder: **CIBA-GEIGY AG**
**Postfach**
**CH-4002 Basel(CH)**

(72) Erfinder: **De Sousa, Bernardo**
**Paradiesstrasse 15**
**CH-4125 Riehen(CH)**

(72) Erfinder: **Gallay, Jean Jacques, Dr.**
**Blumenrain 19**
**CH-4312 Magden(CH)**

(54) **Barbitursäurederivate als Anthelmintika.**

(57) Neue Phenylcarbamoylbarbitursäurederivate der Formel

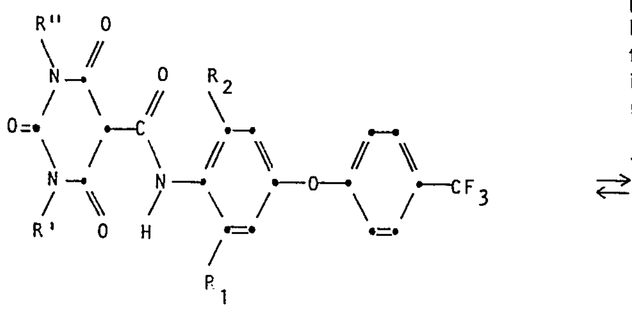

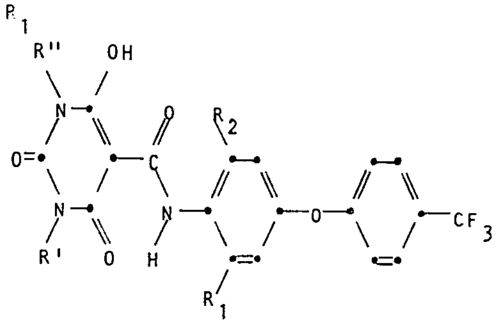

in welcher R', R'', $R_1$ und $R_2$ unabhängig voneinander Wasserstoff, $C_1$-$C_5$-Alkyl, $C_2$-$C_5$-Alkenyl oder den Cyclopropylrest bedeuten, mit der Massgabe, dass die Reste $R_1$ und $R_2$ nicht gleichzeitig Wasserstoff sind, einschliesslich ihrer tautomeren Formen und ihrer Salze, als anthelmintische und insektizide Wirkstoffe. Die Wirkstoffe können in Verbindung mit geeigneten Träger- und weiteren Hilfsstoffen zur Bekämpfung von tier-parasitären Helminthen und keratinvertilgenden Insekten eingesetzt werden.



Croydon Printing Company Ltd.

0102327



CIBA-GEIGY AG BEZEICHNUNG GEÄNDERT siehe Titelseite 5-14068/A
Basel (Schweiz)

## Anthelmintika

Die vorliegende Erfindung betrifft neue 5-Phenylcarbamoylbarbitursäurederivate mit anthelmintischer Wirksamkeit, Mittel, die diese Wirkstoffe als Aktivsubstanzen enthalten sowie die Verwendung der Wirkstoffe bzw. der Mittel zur Bekämpfung von Nematoden, Cestoden und Trematoden in Haus- und Nutztieren, vor allem in Säugetieren.

Die Erfindung betrifft ferner die Herstellung der neuen Wirkstoffe sowie der sie enthaltenden Mittel.

Die neuen Verbindungen entsprechen der allgemeinen Formel I

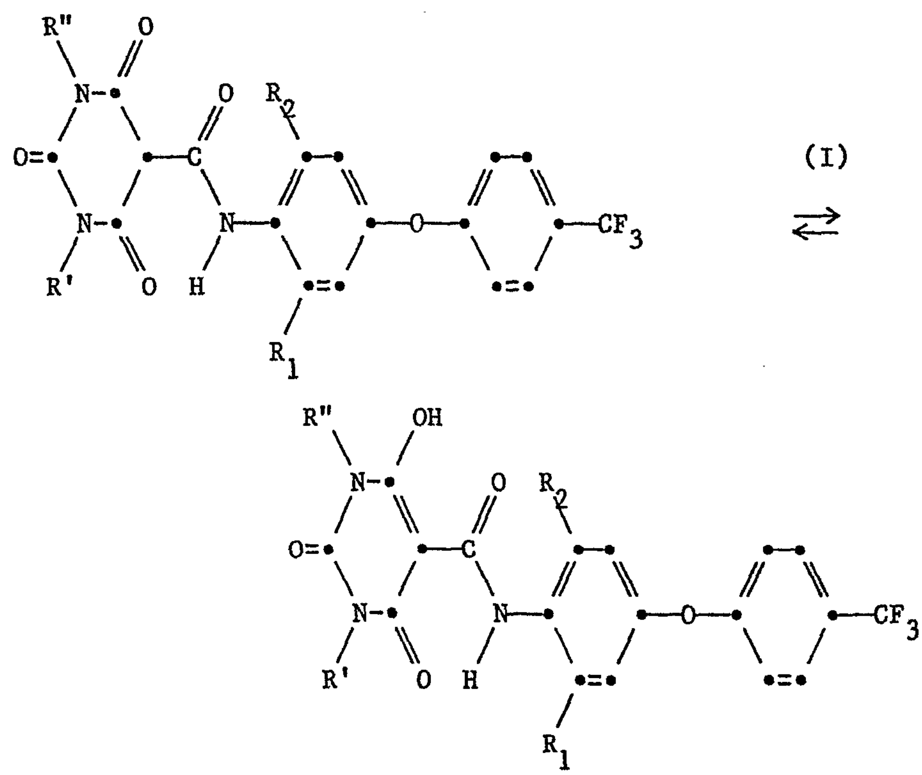

(I)

in welcher R', R", $R_1$ und $R_2$ unabhängig voneinander Wasserstoff, $C_1$-$C_5$-Alkyl, $C_2$-$C_5$-Alkenyl oder den Cyclopropylrest bedeuten, mit der Massgabe, dass die Reste $R_1$ und $R_2$ nicht gleichzeitig Wasserstoff sind, einschliesslich ihrer tautomeren Formen und ihrer Salze.

Zu den betreffenden Salzen zählen beispielsweise die Alkalimetall-, Ammotautomeren Formen und ihrer Salze.

Zu den betreffenden Salzen zählen beispielsweise die Alkalimetall-, Ammonium- oder Aminsalze, wobei Natrium-, Kalium-, Ammonium- oder Alkylaminsalze, besonders Triäthylaminsalze, bevorzugt sind.

Gemäss Formel I sind unter Alkylgruppen gerad- und verzweigtkettige zu verstehen. Dazu zählen die Methyl-, die Aethyl-sowie die Isomeren der Propyl-, der Butyl- und der Pentylgruppe. Alkenylreste sind Vinyl sowie die Isomeren des Propenyls, Butenyls und des Pentenyls. Als bevorzugt sind Verbindungen der Formel I anzusehen, in denen R' oder R" $C_1$-$C_5$-Alkyl bedeutet; ferner Verbindungen in denen R', R", $R_1$ und $R_2$ unabhängig voneinander die Methylgruppe bedeuten oder R' und R" Methyl und gleichzeitig $R_1$ Isopropyl und $R_2$ Wasserstoff darstellen.

5-Phenylcarbamoylbarbitursäurederivate sind in der Europäischen Patentanmeldung Nr. 7,541 als Insektizide sowie als Inhibitoren der Entwicklung von Insekten beschrieben. Sie besitzen folgende allgemeine Formel:

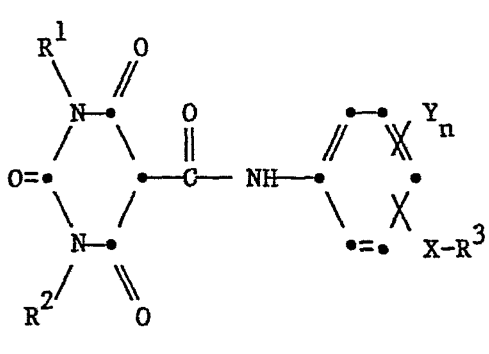

worin $R^1$ = H, Alkyl
$R^2$ = H, Alkyl
$R^3$ = Halogenalkyl, durch Halogenalkyl substituiertes Phenyl
X = O,S
Y = Halogen, Halogenalkyl
n = 0, 1, 2
X, $R^3$, Y = in ortho-Stellung zueinander gemeinsam auch die Gruppe $-O-CF_2-O-CF_2-$ bedeuten.

Die erfindungsgemässen neuen Wirkstoffe der Formel I unterscheiden sich strukturell in charakteristischer Weise von den aus der EP-OS Nr. 7,541 bekannten Barbitursäurederivaten. Dazu wurde überraschenderweise gefunden, dass die neuen Verbindungen ein breites Wirkungsspektrum gegen im Tierorganismus, vor allem in Säugetieren, parasitierende Helminthen besitzen. Sie sind gleichermassen gegen Nematoden, Cestoden und Trematoden mit gutem Erfolg anwendbar. Daneben weisen die Wirkstoffe der Formel I ausgeprägte insektizide Eigenschaften auf, die sie insbesondere zur Bekämpfung von keratinvertilgenden Insekten geeignet machen.

Die Wirkstoffe der Formel I werden hergestellt, indem man zur Reaktion bringt

a) einen Ester der Formel II

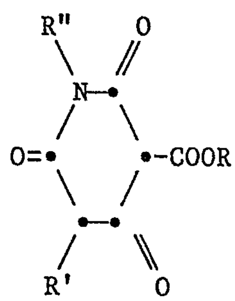

(II)

mit einem Anilinderivat der Formel III

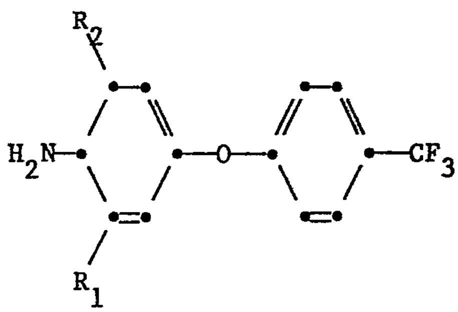

(III)

worin R eine Alkyl- oder eine gegebenenfalls substituierte Phenylgruppe darstellt und die Reste R', R", $R_1$ und $R_2$ die unter der Formel I angegebenen Bedeutungen besitzen,
oder

b) eine substituierte Barbitursäure der Formel IV

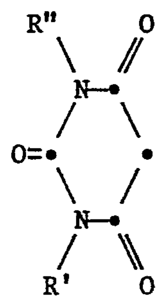

(IV)

mit einem substituierten Phenylisocyanat der Formel (V)

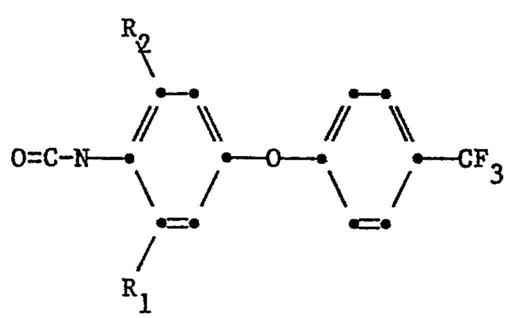

(V)

worin die Reste R', R", $R_1$ und $R_2$ die unter Formel I angegebenen Bedeutungen besitzen,
oder

c) eine substituierte Barbitursäure der Formel IV mit einem substituierten Benzoylazid der Formel VI

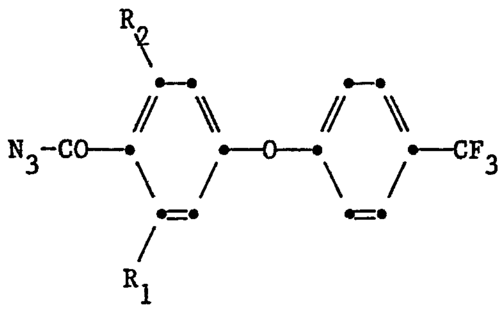

(VI)

worin die Reste R', R", $R_1$ und $R_2$ die unter Formel I angegebenen Bedeutungen besitzen.

Die Herstellungsvarianten (a) und (c) werden bei Reaktionstemperaturen zwischen 80° und 250°C, vorzugweise 100° bis 220°C, durchgeführt. Variante (b) erfordert Reaktionstemperaturen zwischen 0° und 220°C, insbesondere 0° bis 200°C. Die Reaktionen (a), (b) und (c) können bei normalem oder erhöhtem Druck und in Abwesenheit oder vorzugsweise in Gegenwart reaktionsfähiger Lösungs-oder Verdünnungsmittel durchgeführt werden, wobei in manchen Fällen vorteilhafterweise mit einer Base gearbeitet wird.

Die Herstellung der erfindungsgemässen Salze von Verbindungen der Formel I erfolgt durch übliche Neutralisation der freien Säure mit einer Base, insbesondere einer physiologisch verträglichen Base. Vorzugsweise genannt seien Alkalisalze wie Natrium-, Kalium- oder Lithiumsalze sowie Ammoniumsalze und Trialkylaminsalze wie z.B. das bevorzugte Triethylaminsalz. Die Neutralisation wird in einem reaktionsinerten polaren Lösungsmittel, z.B. einem Alkanol, Ester oder einer etherartigen Verbindung, durchgeführt.

Für die Herstellung der erfindungsgemässen Wirksubstanzen geeignete Lösungs- oder Verdünnungsmittel sind z.B. Ether und etherartige Verbindungen wie Dialkylether (Diethylether, Diisopropylether), tert.-Butylmethylether usw.), Anisol, Dioxan, Tetrahydrofuran; aliphatische und aromatische Kohlenwasserstoffe wie Benzol, Toluol,

Petrolether; halogenierte Kohlenwasserstoffe wie Chlorbenzol, Methylenchlorid, Chloroform, Ethylenchlorid, Tetrachlorkohlenstoff, Tetrachlorethylen; Nitrile wie Acetonitril, Propionitril; N,N-dialkylierte Amide wie Dimethylformamid; Dimethylsulfoxid; Ketone wie Aceton, Diethylketon, Methylethylketon und Gemische solcher Lösungsmittel untereinander.

Als Basen kommen organische und anorganischen Basen in Betracht; z.B. vorzugsweise tertiäre Amine wie Trialkylamine (Trimethylamin, Triethylamin, Tripropylamin usw.), Pyridin und Pyridinbasen (z.B. 4-Dimethylaminopyridin, 4-Pyrrolidylaminopyridin usw.), Picoline und Lutidine sowie Oxide, Hydroxide, Carbonate und Hydrogencarbonate von Alkali- und Erdalkalimetallen (z.B. CaO, BaO, NaOH, KOH, $Ca(OH)_2$, $KHCO_3$, $NaHCO_3$, $Ca(HCO_3)_2$, $K_2CO_3$, $Na_2CO_3$ usw.), ferner Acetate wie z.B. $CH_3COONa$ oder $CH_3COOK$. Darüber hinaus eignen sich als Basen auch Alkalialkoholate wie z.B. Natriumethylat, Natriumpropylat, Kalium-tert.-butylat oder Natriummethylat. Die Base wird vorteilhafterweise in bezug auf die Reaktanden in 10 bis 100 % der äquimolaren Menge zugesetzt.

In manchen Fällen kann es von Vorteil sein, wenn die Reaktion unter Schutzgasatmosphäre durchgeführt wird. Geeignete Schutzgase sind z.B. Stickstoff, Helium, Argon oder Kohlendioxid.

Die freie Säure der Formel I führt durch Umsetzung mit Basen zu den ebenfalls zur Erfindung gehörenden Salzen.

Die in den Herstellungsvarianten (a), (b) und (c) genannten Ausgangsstoffe sind bekannt (siehe z.B. Chem. Ber. 54, 1038 [1921]) oder können in analoger Weise wie die bekannten Substanzen hergestellt werden.

Das beschriebene Herstellungsverfahren ist einschliesslich aller Varianten (a), (b) und (c) ein Bestandteil vorliegender Erfindung.

Die erfindungsgemässen Wirkstoffe der Formel I können in unterschiedlichen tautomeren Formen vorliegen, nämlich in der Keto- oder Enolform oder in einem Gemisch aus Keto- und Enolform. Diese Erfindung betrifft sowohl die einzelnen Tautomeren als auch deren Gemische, aber auch die Salze jeder dieser Formen und deren Herstellung.

Die Erfindung schliesst auch ein Verfahren zum Schutz von Tieren vor parasitären Helminthen ein, das dadurch gekennzeichnet ist, dass man die Wirkstoffe der Formel I bzw. die Wirkstofformulierungen als Zusatz zum Futter oder zu den Tränken oder auch in fester oder flüssiger Form oral, durch Injektion oder mittels der Pour-on-Methode den Tieren appliziert. Darüber hinaus betrifft die Erfindung auch die Verwendung der Wirkstoffe der Formel I zur Bekämpfung von Insekten, insbesondere von keratinvertilgenden Insekten. Diese Verwendung besteht im einzelnen in einem Verfahren zum Schutz von keratinischen bzw. keratinhaltigen Materialien vor dem Befall durch keratinfressende Insekten und vor Insektenfrass, das dadurch gekennzeichnet ist, dass man das zu schützende Material mit dem Wirkstoff oder einer Wirkstofformulierung behandelt. Ferner betrifft die Erfindung die Wirkstoffe der Formel I bzw. sie enthaltende geeignete Formulierungen als Schutzmittel für keratinisches bzw. keratinhaltiges Material gegen keratinfressende Insekten sowie das mit Hilfe von Verbindungen der Formel I geschützte bzw. ausgerüstete Material.

In jedem der erfindungsgemässen Verfahren zur Schädlingsbekämpfung bzw. der erfindungsgemässen Schädlingsbekämpfungsmittel können die Wirkstoffe der Formel I in allen tautomeren Formen, deren Mischungen oder in Form ihrer Salze eingesetzt werden.

Unter den bei Warmblütern vorkommenden Endoparasiten verursachen namentlich die Helminthen grosse Schäden. So zeigen zum Beispiel von diesen Parasiten befallene Tiere nicht nur ein verlangsamtes Wachstum, sonderen teilweise sogar Schädigungen, die zum Absterben führen. Daher ist es von grosser Bedeutung, therapeutische

Mittel zu entwickeln, die sich zur Bekämpfung von Helminthen und deren Entwicklungsstadien, sowie zur Vorbeugung gegen den Befall durch diese Parasiten eignen. Besonders gefährliche Wurmkrankheiten sind solche, die durch im Magen-Darmtrakt und anderen Organen parasitierende Nematoden, Cestoden und Trematoden hervorgerufen werden und vor allem bei Wiederkäuern, wie Schafen, Rindern und Ziegen, sowie Equiden und Geflügel auftreten.

Die durch Helminthiasen verursachten Schäden können bei chronischem und vor allem bei epidemischem Auftreten der Infestation in Viehherden beträchtlich sein. Sie äussern sich unter anderem in Produktivitäts-Verminderungen, geschwächter Widerstandskraft und erhöhter Mortalität. Bekämpfung und Vorbeugung von Helminthiasen gelten deshalb als vordringliche Aufgabe, um derartige vor allem volkswirtschaftlich ins Gewicht fallende Schäden zu vermeiden oder zu mindern.

In der vorliegenden Beschreibung werden unter dem Begriff "Helminthen" insbesondere parasitische Würmer verstanden, die zu den Phyla Platyhelminthes (Cestoden, Trematoden) und Nemathelminthes (Nematodes und Verwandte) gehören, also Bandwürmer, Saugwürmer und Rundwürmer des Gastrointestinal-Traktes und anderer Organe (z.B. Leber, Lunge, Niere, Lymphgefässe, Blut etc.). Es sind zwar eine Reihe von Stoffen mit anthelmintischer Wirkung bekannt, die für die Bekämpfung der verschiedenen Helminthen species vorgeschlagen wurden. Diese vermögen jedoch nicht voll zu befriedigen, sei es, dass bei verträglicher Dosierung eine Ausschöpfung ihres Wirkungsspektrums nicht möglich ist, oder dass sie in therapeutisch wirksamen Dosen unerwünschte Nebenwirkungen oder Eigenschaften zeigen. In diesem Zusammenhang spielt auch die heute vermehrt auftretende Resistenz gegen bestimmte Stoffklassen eine immer bedeutendere Rolle. Das beispielsweise in der Literatur beschriebene "Albendazol" (British Pat. No. 1464326; Am. J. Vet. Res. 38, 1425-1426 (1977); Am. J. Vet. Res. 37, 1515-1516 (1976); Am. J. Vet. Res. 38, 807-808 (1977); Am. J. Vet. Res. 38, 1247-1248 (1977)) besitzt als bekanntes Anthelmintikum zwar ein Wirkungsspektrum bei Wiederkäuern, jedoch ist z.B.

seine Wirkung gegen Benzimidazol-resistente Nematoden und adulte Leberegel völlig unzureichend, wobei vor allem die pathologisch wichtigen unreifen Wanderformen der letzteren bei den für das Wirtstier verträglichen Dosierungen nicht angegriffen werden.

Ueberraschenderweise wurde nun festgestellt, dass die Wirkstoffe der Formel I sowohl eine intensive anthelmintische Wirksamkeit mit breitem Wirkungsspektrum gegen Nematoden, Cestoden und Trematoden als auch zusätzlich eine günstige Warmblütertoxizität besitzen.

Die erfindungsgemässen neuen Wirkstoffe der Formel I sind beispielsweise zur Bekämpfung parasitärer Nematoden der Ordnungen (nach K.I. Skrajabin)

Rhabditida
Ascaridida
Spirurida
Trichocephalida

oder zur Bekämpfung von Cestoden der Ordnungen (nach Wardle & McLeod)

Cyclophyllidae
Pseudophyllidae

oder zur Bekämpfung von Trematoden der Ordnung

Digenea

bei Haus- und Nutztieren, wie Rindern, Schafen, Ziegen, Pferden, Schweinen, Katzen, Hunden und Geflügel, geeignet. Sie können den Tieren sowohl als Einzeldosis als auch wiederholt verabreicht werden, wobei die einzelnen Gaben je nach Tierart vorzugsweise zwischen 1 und 100 mg pro kg Körpergewicht betragen. Durch eine protrahierte Verabreichung erzielt man in manchen Fällen eine bessere Wirkung oder man kann mit geringeren Gesamtdosen auskommen.

Die erfindungsgemäsen Mittel werden hergestellt, indem die Wirkstoffe der Formel I mit flüssigen und/oder festen Formulierungshilfsstoffen durch schrittweises Vermischen und/oder Vermahlen derart in

Kontakt gebracht werden, sodass eine anwendungskonforme optimale Entfaltung der anthelmintischen Aktivität der Formulierung erzielt wird.

Die Formulierungsschritte können durch Kneten, Granulieren (Granulate) und gegebenenfalls Pressen (Pellets) ergänzt werden.

Als Formulierungshilfsstoffe dienen beispielsweise feste Trägerstoffe, Lösungsmittel und gegebenenfalls oberflächenaktive Stoffe (Tenside).

Zur Bereitung der erfindungsgemässen Mittel werden folgende Formulierungshilfsstoffe verwendet:

Feste Trägerstoffe wie z.B. Kaolin, Talkum, Bentonit, Kochsalz, Calciumphosphat, Kohlenhydrate, Cellulosepulver, Baumwollsaatmehl, Polyäthylenglykoläther, gegebenenfalls Bindemittel wie z.B. Gelatine, lösliche Cellulosederivate, gewünschtenfalls unter Zusatz von oberflächenaktiven Stoffen wie ionischen oder nicht-ionischen Dispersionsmitteln; ferner natürliche Gesteinsmehle wie Calcit, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinertes Pflanzenmaterial verwendet werden.

Als Lösungsmittel kommen in Frage: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat; aliphatische Kohlenwasserstoffe wie z.B. Cyclohexan oder Paraffine, Alkohole und Glykole sowie deren Ether und Ester, wie z.B. Ethanol, Ethylenglykol, Ethylenglykolmonomethyl- oder

Ethylether, Ketone wie z.B. Cyclohexanon, stark polare Lösungsmittel wie z.B. N-Methyl-2-pyrrolidon, Dimethylsulfoxid oder Dimethylformamid, sowie gegebenenfalls epoxydierte Pflanzenöle wie z.B. epoxydiertes Kokosnussöl oder Sojaöl und Wasser.

Als oberflächenaktive Verbindungen kommen je nach Art des zu formulierenden Wirkstoffes der Formel I nicht-ionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-methyllaurinsalze zu erwähnen.

Häufig werden sog. synthetische Tenside verwendet, insbesondere Fettsulfonate, Fettsulfate, sulfonierte Benzimidazolderivate oder Alkylsulfonate.

Die Fettsulfonate oder -sulfate liegen in der Regel als Alkali-, Erdalkali- oder gegebenenfalls substituierte Ammoniumsalze vor und weisen einen Alkalirest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B.

die Na-, Ca- oder Triethanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxyd-Adduktes in Frage.

Als nicht-ionische Tenside kommen in erster Linie Polyglykoletherderivate von aliphatischen oder cycloaliphatischen Alkoholen, gesättigten oder ungesättigten Fettsäuren und Alkyphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nicht-ionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als

Halogenide, Methylsulfate oder Ethylsulfate vor, z.B. das Stearyltrimethylammoniumchlorid oder das Benzyldi(2-chlorethyl)ethylammoniumbromid.

Die in der Formulierungstechnik gebräuchlichen Tenside sind u.a. in folgenden Publikationen beschrieben:

"Mc Cutcheon's Detergents and Emulsifiers Annual" BC Publishing Corp., Ringwood New Jersery, 1980;
Sisely and Wood, "Encyclopedia of Surface Active Agents", Chemical Publishing Co., Inc. New York, 1980.

Als Bindemittel für Tabletten und Boli kommen chemisch abgewandelte, in Wasser oder Alkohol lösliche, polymere Naturstoffe in Frage, wie Stärke-, Cellulose- oder Protein-derivate (z.B. Methylcellulose, Carboxymethylcellulose, Ethylhydroxyethylcellulose, Proteine wie Zein, Gelatine und dergleichen) sowie synthetische Polymere wie z.B. Polyvinylalkohol, Polyvinylpyrrolidon etc.. Ferner sind in Tabletten Füllstoffe, (z.B. Stärke, mikrokristalline Cellulose, Zucker, Milchzucker etc.), Gleitmittel und Sprengmittel enthalten.

Liegen die anthelmintischen Mittel in Form von Futterkonzentraten vor, so dienen als Trägerstoffe z.B. Leistungsfutter, Futtergetreide oder Proteinkonzentrate. Solche Futterkonzentrate oder -mittel können ausser den Wirkstoffen noch Zusatzstoffe, Vitamine, Antibiotika, Chemotherapeutika, oder andere Pestizide, vornehmlich Bakteriostatika, Fungistatika, Coccidiostatika, oder auch Hormonpräparate, Stoffe mit anaboler Wirkung oder das Wachstum begünstigende, die Fleischqualität von Schlachttieren beeinflussende oder in anderer Weise für den Organismus nützliche Stoffe enthalten. Werden die Mittel oder die darin enthaltenen Wirkstoffe der Formel I direkt dem Futter oder den Viehtränken zugesetzt, so enthält das Fertigfutter oder die Fertigtränke die Wirkstoffe vorzugsweise in einer Konzentration von etwa 0,0005 bis 0,02 Gewichtsprozent (5-200 ppm).

Die Applikation der erfindungsgemässen Mittel an die zu behandelnden Tiere kann peroral, parenteral, subcutan oder topikal durchgeführt werden, wobei die Mittel in Form von Lösungen, Emulsionen, Suspensionen (Drenchs), Pulvern, Tabletten, Bolussen und Kapseln vorliegen.

Die erfindungsgemässen anthelmintischen Mittel enthalten in der Regel 0,1 bis 99 Gew.%, insbesondere 0,1 bis 95 Gew.-% Wirkstoff der Formel I, 99,9 bis 1 Gew.-%, insbesondere 99,8 bis 5 Gew.-%, eines festen oder flüssigen Zusatzstoffes, darunter 0 bis 25 Gew.-%, insbesondere 0,1 bis 25 Gew.-%, eines Tensides.

Während als Handelsware eher konzentrierte Mittel bevorzugt werden, verwendet der Endverbraucher in der Regel verdünnte Mittel.

Solche Mittel können noch weitere Zusätze wie Stabilisatoren, Entschäumer, Viskositätsregulatoren, Bindemittel, Haftmittel sowie andere Wirkstoffe zur Erzielung spezieller Effekte enthalten.

Derartige vom Endverbraucher verwendete anthelmintische und/oder insektizide Mittel sind ebenfalls ein Bestandteil der vorliegenden Erfindung.

Die nachfolgenden Beispiele dienen zur näheren Erläuterung der Erfindung, ohne dieselbe einzuschränken.

Herstellungsbeispiele:

Beispiel 1: Herstellung von 1,3-Dimethyl-5-[2,6-dimethyl-4-(4-trifluormethylphenoxy)-phenylcarbamoyl]-barbitursäure

13,7 g (0,06 Mol) 1,3-Dimethyl-5-ethoxycarbonyl-barbitursäure und 16,9 g (0,06 Mol) 2,6-Dimethyl-4-(4-trifluormethylphenoxy)-anilin werden miteinander gut vermischt und unter Schutzgasatmosphäre (Stickstoff) 1 Stunde auf 160°C (Aussentemperatur) erhitzt, wobei Ethanol entweicht. Zur Vervollständigung der Reaktion wird der

Kolbeninhalt weitere 10 Minuten auf 180°C (Aussentemperatur) erhitzt, anschliessend auf Raumtemperatur abgekühlt und 2 mal aus Toluol/Hexan (1:1) umkristallisiert.

Smp. 189-191°C Ausbeute 19,6 g (= 71 % d. Th.)

Beispiel 2: Herstellung von 1,3-Dimethyl-5-[2-isopropyl-4-(4-trifluormethylphenoxy)-phenylcarbamoyl]-barbitursäure

9,1 g (0,04 Mol) 1,3-Dimethyl-5-ethoxycarbonyl-barbitursäure und 11,8 g (0,04 Mol) 2-Isopropyl-4-(4-trifluormethylphenoxy)-anilin werden miteinander gut vermischt und unter Schutzgasatmosphäre (Stickstoff) 1 Stunde auf 160°C (Aussentemperatur) erhitzt, wobei Ethanol entweicht. Zur Vervollständigung der Reaktion wird der Kolbeninhalt weitere 10 Minuten auf 180°C (Aussentemperatur) erhitzt, anschliessend auf Raumtemperatur abgekühlt und 2 mal aus Ligroin umkristallisiert.

Smp. 158-159°C Ausbeute 11,8 g (= 61,8 % d.Th.)

Tabelle I

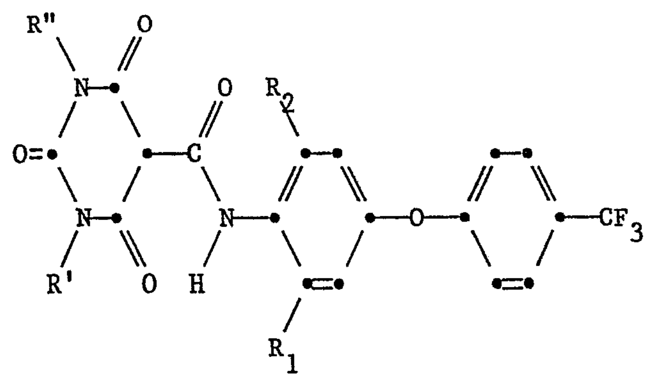

| Nr. | R' | R" | $R_1$ | $R_2$ | Schmelzpunkt in °C |
|---|---|---|---|---|---|
| 1 | $-CH_3$ | $-CH_3$ | $-CH(CH_3)_2$ | H | 158-159 |
| 2 | $-CH_3$ | $-CH_3$ | $-CH_3$ | $-CH_3$ | 189-191 |
| 3 | $-C_2H_5$ | $-C_2H_5$ | $-CH(CH_3)_2$ | H | |
| 4 | $-CH_3$ | $-CH_3$ | $-CH_3$ | H | 163-165 |
| 5 | $-CH_2CH=CH_2$ | $-CH_3$ | $-CH_3$ | H | 139-140 |

| Nr. | R' | R" | $R_1$ | $R_2$ | Schmelz-punkt in °C |
|---|---|---|---|---|---|
| 6 | 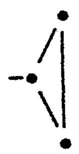 | 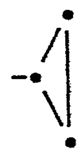 | $-CH_3$ | $-CH_3$ | |
| 7 | $-C_2H_5$ | $-C_2H_5$ | $-CH_3$ | $-CH_3$ | 144-146 |
| 8 | $-C(CH_3)_3$ | $-C(CH_3)_3$ | $-CH_3$ | $-CH_3$ | |
| 9 | $-C_2H_5$ | H | $-CH_3$ | $-CH_3$ | |
| 10 | $-CH_3$ | H | $-CH_3$ | $-CH_3$ | |
| 11 | 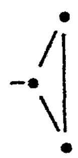 | $-CH_3$ | $-CH_3$ | $-CH_3$ | 147-149 |
| 12 | $-CH_2CH=CH_2$ | $-CH_3$ | $-CH_3$ | $-CH_3$ | 139-140 |
| 13 | 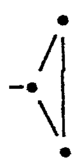 | $-CH_3$ | $-CH(CH_3)_2$ | H | 133-134 |
| 14 | $-CH_2CH=CH_2$ | $-CH_3$ | $-CH(CH_3)_2$ | H | |
| 15 | $-CH_3$ | $-CH_3$ | $-C_2H_5$ | H | |
| 16 | $-C_2H_5$ | $-C_2H_5$ | $-C_2H_5$ | $-C_2H_5$ | |
| 17 | $-CH_3$ | $-CH_3$ | $-C_2H_5$ | $-C_2H_5$ | |

Formulierungsbeispiele (% = Gewichtsprozent)

| 3. Emulsions-Konzentrate | a) | b) | c) |
|---|---|---|---|
| Wirkstoff aus Tabelle I | 25% | 40% | 50% |
| Ca-Dodecylbenzolsulfonat | 5% | 8% | 6% |
| Ricinusöl-polyethylenglykolether (36 Mol Ethylenoxid) | 5% | - | - |
| Tributylphenol-polyethylenglykolether (30 Mol Ethylenoxid) | - | 12% | 4% |

| | | | |
|---|---|---|---|
| Cyclohexanon | - | 15% | 20% |
| Xylolgemisch | 65% | 25% | 20% |

Aus solchen Konzentraten können durch Verdünnen mit Wasser Emulsionen jeder gewünschten Konzentration hergestellt werden.

| 4. Lösungen | a) | b) | c) | d) |
|---|---|---|---|---|
| Wirkstoff aus Tabelle I | 80% | 10% | 5% | 95% |
| Ethylenglykol-monomethyl-ether | 20% | - | - | - |
| Polyethylenglykol MG 400 | - | 70% | - | - |
| N-Methyl-2-pyrrolidon | - | 20 | - | - |
| Epoxydiertes Kokosnussöl | - | - | 1% | 5% |
| Benzin (Siedegrenze 160-190°C) | - | - | 94% | - |

(MG = Molekulargewicht)

Die Lösungen sind zur Anwendung in Form kleinster Tropfen geeignet.

| 5. Granulate | a) | b) |
|---|---|---|
| Wirkstoff der Tabelle I | 5% | 10% |
| Kaolin | 94% | - |
| Hochdisperse Kieselsäure | 1% | - |
| Attapulgit | - | 90% |

Der Wirkstoff wird in Methylenchlorid gelöst, auf den Träger aufgesprüht und das Lösungsmittel anschliessend im Vakuum eingedampft. Solche Granulate können dem Viehfutter beigemischt werden.

| 6. Stäubemittel | a) | b) |
|---|---|---|
| Wirkstoff der Tabelle I | 2% | 5% |
| Hochdisperse Kieselsäure | 1% | 5% |
| Talkum | 97% | - |
| Kaolin | - | 90% |

Durch inniges Vermischen auf Trägerstoffe mit dem Wirkstoff erhält man gebrauchsfertige Stäubemittel.

| 7. In Wasser dispergierbare Pulvermischung | a) | b) | c) |
|---|---|---|---|
| Wirkstoff aus Tabelle I | 25% | 50% | 75% |
| Na-Ligninsulfonat | 5% | 5% | - |
| Oelsäure | 3% | - | 5% |
| Na-Diisobutylnaphthalinsulfonat | - | 6% | 10% |
| Octylphenolpolyethylenglykolether (7-8 Mol Ethylenoxid) | - | 2% | - |
| Hochdisperse Kieselsäure | 5% | 10% | 10% |
| Kaolin | 62% | 27% | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 8. Emulsions-Konzentrat | a) | b) | c) |
|---|---|---|---|
| Wirkstoff aus Tabelle I | 10% | 8% | 60% |
| Octylphenolpolyethylenglykolether 4-5 Mol Ethylenoxid) | 3% | 3% | 2% |
| Ricinusölpolyglykolether (35 Mol Ethylenoxid) | 4% | 5% | 4% |
| Cyclohexanon | 30% | 40% | 15% |
| Xylolgemisch | 50% | 40% | 15% |

Aus diesem Konzentrat können durch Verdünnen mit Wasser Emulsionen jeder gewünschten Konzentration hergestellt werden.

| 9. Stäubemittel | a) | b) |
|---|---|---|
| Wirkstoff aus Tabelle I | 5% | 8% |
| Talkum | 95% | - |
| Kaolin | - | 92% |

Man erhält anwendungsfertige Stäubemittel, indem der Wirkstoff mit dem Träger vermischt auf einer geeigneten Mühle vermahlen wird.

10. Granulat

| | |
|---|---|
| Wirkstoff aus Tabelle I | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrom getrocknet.

11. Granulat

| | |
|---|---|
| Wirkstoff aus Tabelle I | 3% |
| Polyethylenglykol (MG 200) | 3% |
| Kaolin | 94% |

(MG = Molekulargewicht)

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

12. Suspensions-Konzentrat

| | |
|---|---|
| Wirkstoff aus Tabelle I | 40% |
| Ethylenglykol | 10% |
| Nonylphenolpolyethylenglykolether (15 Mol Ethylenoxid) | 6% |
| N-Ligninsulfonat | 10% |
| Carboxymethylcellulose | 1% |
| 37%ige wässrige Formaldehyd-Lösung | 0,2% |
| Silikonöl in Form einer 75%igen wässrigen | |

| | |
|---|---|
| Emulsion | 0,8% |
| Wasser | 32% |

Der fein gemahlene Wirkstoff wird mit den Zusatzstoffen innig vermischt. Man erhält so ein Suspension-Konzentrat, aus welchem durch Verdünnen mit Wasser Suspensionen jeder gewünschten Konzentration hergestellt werden können.

Beispiel 13: Tabletten bzw. Boli

| | | |
|---|---|---|
| I | Wirkstoff der Tabelle I | 33,0% |
| | Methylcellulose | 0,80% |
| | Kieselsäure hochdispers | 0,80% |
| | Maisstärke | 8,40% |
| II | Milchzucker krist. | 22,50% |
| | Maisstärke | 17,00% |
| | mikrokrist. Cellulose | 16,50% |
| | Magnesiumstearat | 1,00% |

I Methylcellulose in Wasser einrühren und quellen lassen; Kieselsäure in die Quellung einrühren und homogen suspendieren. Wirkstoff und Maisstärke mischen. In diese Mischung die wässrige Suspension einarbeiten und zu einem Teig kneten. Diese Masse durch ein Sieb 12 M granulieren und trocknen.

II Alle 4 Hilfsstoffe gut mischen.

III Phasen I und II mischen und zu Tabletten oder Boli verpressen.

Biologische Beispiele

Die anthelmintische Wirksamkeit wird anhand folgender Versuche demonstriert:

Beispiel 14: Versuch an mit Nematoden wie Haemonchus concortus und Trichostrongylus colubriformis infizierten Schafen

Der Wirkstoff wird in Form einer Suspension mit einer Magensonde oder durch Pansen-Injektion Schafen verabreicht, die vorher mit Nematoden wie Haemonchus concortus und Trichostrongylus colubriformis künstlich infiziert wurden. Pro Versuch resp. pro Dosis werden 1 bis 3 Tiere verwendet. Jedes Schaf wird mit nur einer einzigen Dosis behandelt.

Eine erste Evaluierung erfolgt dadurch, dass die Anzahl der vor und nach der Behandlung im Kot der Schafe ausgeschiedenen Wurmeier verglichen werden.

Sieben bis zehn Tage nach der Behandlung werden die Schafe getötet und seziert. Die Auswertung erfolgt durch Auszählung der nach der Behandlung im Darm zurückbleibenden Würmer. Gleichzeitig und gleichartig infizierte aber unbehandelte Schafe dienen als Kontrolle resp. Vergleich.

Schafe, die mit einer Suspension eines Wirkstoffes aus der Tabelle I behandelt werden, zeigen im Vergleich zu unbehandelten, aber infizierten Vergleichsgruppen einen um mindestens 95 % reduzierten Nematodenbefall. Die Wirkungsdosis beträgt 20, 10, 5 und 2 mg/kg. Mit den Verbindungen Nr. 1, 2, 4, 11, 12 und 13 wird bei jeder der Dosen 100 % Wirkung erzielt.

Beispiel 15: Versuch an mit Cestoden wie Moniezia benedeni infizierten Schafen

Der Wirkstoff wird in Form einer Suspension mit einer Magensonde oder durch Pansen-Injektion Schafen verabreicht, die vorher mit Cestoden wie Moniezia benedeni infiziert wurden. Pro Versuch resp. pro Dosis werden 3 Tiere verwendet. Jedes Schaf wird mit nur einer einzigen Dosis behandelt.

Sieben bis zehn Tage nach der Behandlung werden die Schafe getötet und seziert. die Auswertung erfolgt durch Auszählung der nach der Behandlung im Darm zurückbleibenden Würmer. Gleichzeitig und gleichartig infizierte aber unbehandelte Schafe dienen als Kontrolle resp. Vergleich. In diesem Versuch bewirken Wirkstoffe aus der Tabelle I bei den angewandten Dosen von 15, 10, 5 und 2 mg/kg Körpergewicht eine vollständige Reduktion des Cestodenbefalls (100 % Wirkung).

Beispiel 16: Versuch an mit Fasciola hepatica infizierten Schafen

Der Wirkstoff wird in Form einer Suspension mit einer Magensonde oder durch Pansen-Injektion Schafen verabreicht, die vorher mit Fasciola hepatica künstlich infiziert wurden. Pro Versuch resp. pro Dosis werden 3 Tiere verwendet. Jedes Tier wird mit nur einer einzigen Dosis behandelt.

Eine erste Evaluierung erfolgt dadurch, dass die Anzahl der vor und nach der Behandlung im Kot der Schafe ausgeschiedenen Wurmeier verglichen werden.

Drei bis vier Wochen nach der Behandlung werden die Schafe getötet und seziert. Die Auswertung erfolgt durch das Auszählen der nach der Behandlung in den Gallengängen zurückgebliebenen Leberegel. Gleichzeitig und gleichartig infizierte aber unbehandelte Schafe dienen als Kontrolle resp. Vergleich. Der Unterschied der in den beiden Gruppen festgestellten Anzahl von Leberegeln ergibt den Wirkungsgrad des geprüften Wirkstoffs.

In diesem Versuch zeigen Wirkstoffe aus der Tabelle I bei Dosen von 30 und 20 mg/kg Körpergewicht stets eine mindestens 95%ige Wirksamkeit gegen Fasciola hepatica. Die Verbindungen Nr. 1, 2, 4, 5, 11, 12 und 13 zeigen jeweils vollständige Wirksamkeit (= Abtötung 100 %).

Patentansprüche

1. 5-Phenylcarbamoylbarbitursäurederivate der allgemeinen Formel I

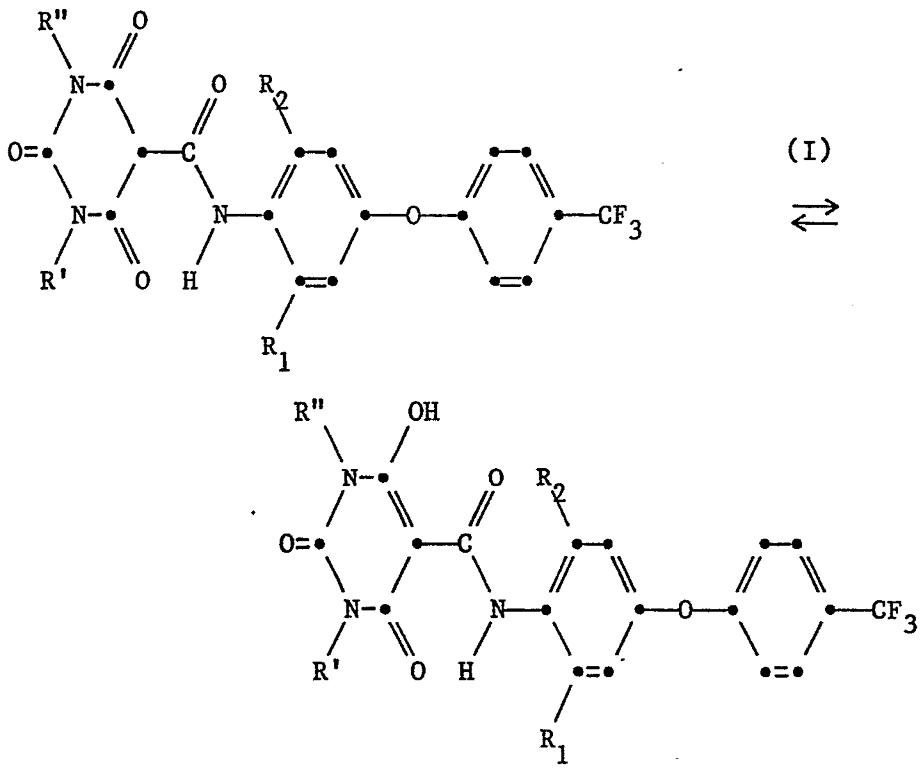

(I)

in welcher R', R", $R_1$ und $R_2$ unabhängig voneinander Wasserstoff, $C_1$-$C_5$-Alkyl, $C_2$-$C_5$-Alkenyl oder den Cyclopropylrest bedeuten mit der Einschränkung, dass die Reste $R_1$ und $R_2$ nicht gleichzeitig Wasserstoff sind, einschliesslich ihrer tautomeren Formen und ihrer Salze.

2. Verbindungen nach Anspruch 1 gemäss Formel I, in welchen R' und R" $C_1$-$C_5$-Alkyl bedeuten und $R_1$ und $R_2$ die unter Formel I angegebenen Bedeutungen besitzen.

3. Verbindungen nach Anspruch 1 gemäss Formel I, in welchen R', R", $R_1$ und $R_2$ unabhängig voneinander die Methylgruppe bedeuten oder R' und R" Methyl und gleichzeitig $R_1$ Isopropyl und $R_2$ Wasserstoff darstellen.

4. 1,3-Dimethyl-5-[2,6-dimethyl-4-(4-trifluormethylphenoxy)-phenylcarbamoyl]-barbitursäure.

5. 1,3-Dimethyl-5-[2-isopropyl-4-(4-trifluormethylphenoxy)-phenylcarbamoyl]-barbitursäure.

6. Verfahren zur Herstellung von Verbindungen der Formel I gemäss Anspruch 1, dadurch gekennzeichnet, dass man

a) bei Temperaturen von 80° bis 250°C einen Ester der Formel

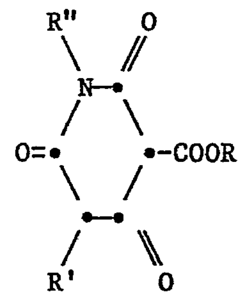

(II)

mit einem Anilinderivat der Formel III

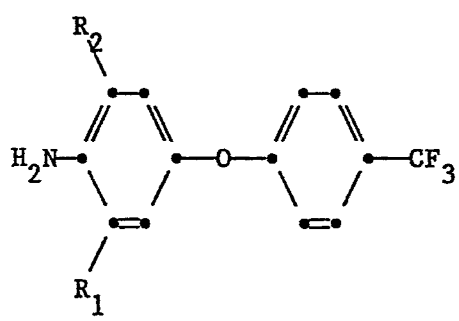

(III)

worin R eine Alkyl- oder eine gegebenenfalls substituierte Phenylgruppe darstellt und die Reste R', R", $R_1$ und $R_2$ die unter der Formel I in Anspruch 1 angegebenen Bedeutungen besitzen, umsetzt oder

b) bei Temperaturen von 0° bis 220°C eine substituierte Barbitursäure der Formel IV

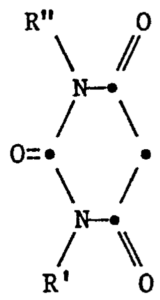

(IV)

mit einem substituierten Phenylisocyanat der Formel (V)

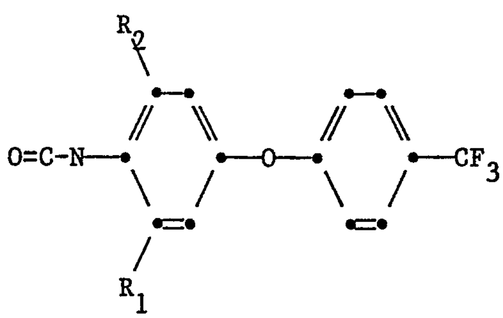

(V)

worin die Reste R', R", $R_1$ und $R_2$ die unter Formel I in Anspruch 1 angegebenen Bedeutungen besitzen, umsetzt
oder

c) bei Temperaturen von 80° bis 250°C eine substituierte Barbitursäure der Formel IV

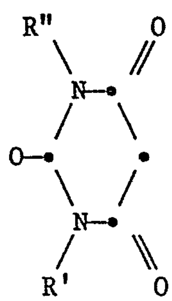

(IV)

mit einem substituierten Benzoylazid der Formel

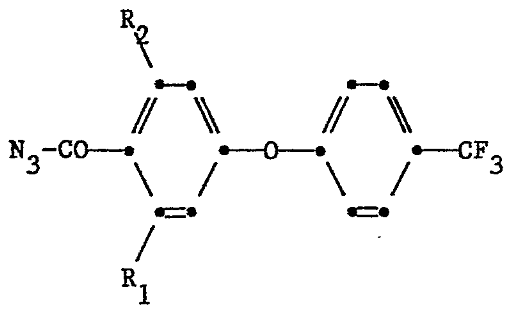

(VI)

worin die Reste R', R", $R_1$ und $R_2$ die unter Formel I in Anspruch 1 angegebenen Bedeutungen besitzen, umsetzt.

7. Verfahren nach Anspruch 6, dadurch gekennzeichnet, dass die Verfahren (a), (b) und (c) in Gegenwart reaktionsinerter Lösungs- oder Verdünnungsmittel durchgeführt werden.

8. Verfahren nach den Ansprüchen 6 und 7, dadurch gekennzeichnet, dass die Verfahren (a) und (c) bei 100° bis 220°C und Verfahren (b) bei 0° bis 200°C durchgeführt werden.

9. Verfahren nach den Ansprüchen 7 bis 8, dadurch gekennzeichnet, dass die Verfahren (a), (b) und (c) in Gegenwart einer Base durchgeführt werden.

10. Anthelmintisches Mittel, dadurch gekennzeichnet, dass es als aktive Komponente mindestens eine Verbindung der Formel I, ein Tautomeres oder ein Salz davon gemäss Anspruch 1 neben Träger- und weiteren Hilfsstoffen enthält.

11. Insektizides Mittel gegen keratinvertilgende Insekten, dadurch gekennzeichnet, dass es als aktive Komponente mindestens eine Verbindung der Formel I, ein Tautomeres oder ein Salz davon gemäss Anspruch 1 neben Träger- und weiteren Hilfsstoffen enthält.

12. Mittel nach Ansprüchen 10 und 11, dadurch gekennzeichnet, dass es einen Wirkstoffgehalt von 0,1 bis 99,0 Gew.-% und einen Gehalt an Träger- und weiteren Hilfsstoffen von 99,9 bis 1 Gew.-% besitzt.

13. Verfahren zur Bekämpfung parasitärer Helminthen, dadurch gekennzeichnet, dass man einem Tier eine anthelmintisch wirksame Menge einer Verbindung der Formel I gemäss Anspruch 1 verabreicht.

14. Verwendung einer Verbindung der Formel I gemäss Anspruch 1 zur Bekämpfung von parasitären Helminthen.

15. Verwendung einer Verbindung der Formel I gemäss Anspruch 1 zur Bekämpfung von keratinvertilgenden Insekten.